# FASCICULE DE BREVET EUROPEEN

(11) **EP 1 592 319 B1**
(45) Date de publication et mention de la délivrance du brevet: **31.10.2007**
(21) Numéro de dépôt: 04707247.5
(22) Date de dépôt: 02.02.2004
(51) Int. Cl.: A45D 1/00

(54) **COMPOSITION COSMETIQUE COMPRENANT UN AMIDE IONIQUE HYDROGELIFIANT ET UN POLYMERE BENEFIQUE POUR LA CHEVELURE**
KOSMETISCHE ZUSAMMENSETZUNG ENTHALTEND EIN IONISCHES HYDROGELBILDENDES AMID UND EIN HAARPFLEGENDES POLYMER
COSMETIC COMPOSITION COMPRISING A HYDROGELLING IONIC AMIDE AND A POLYMER BENEFICIAL TO THE HAIR

(30) Priorité: 03.02.2003 FR 0350010; 10.06.2003 US 477046 P
(43) Date de publication de la demande: 09.11.2005
(73) Titulaire: L'ORÉAL, 75008 Paris (FR)
(72) Inventeur: ROLLAT-CORVOL, Isabelle, F-75017 Paris (FR); BENABDILLAH, Katarina, 95130 Le Plessis-Bouchard (FR)
(74) Mandataire: Bourdeau, Françoise
(86) Numéro de dépôt international: PCT/FR2004/000226
(87) Numéro de publication internationale: WO 2004/068996

(56) Documents cités:
- EP-A- 1 197 206
- WO-A-99/15610
- MASAHIRO SUZUKI ET AL.: "Low molecular-weight hydrogelators based on L-lysine with various pyridinium cations" CHEMISTRY LETTERS, 2002, pages 704-705, XP009018287 cité dans la demande
- MASAHIRO SUZUKI ET AL.: "New low-molecular-weight hydrogelators based on L-lysine with positively charged pendant chain" NEW JOURNAL OF CHEMISTRY, vol. 26, 2002, pages 817-818, XP009018282 cité dans la demande
- MASAHIRO SUZUKI ET AL.: "Novel family of low molecular weight hydrogelators based on L-lysine derivatives" CHEMICAL COMMUNICATIONS, 2002, pages 884-885, XP009018628 cité dans la demande

## Description

L'invention a pour objet une composition cosmétique comprenant au moins un polymère bénéfique et au moins un amide ionique hydrogélifiant. Elle vise également un procédé cosmétique comprenant l'application de cette composition, ainsi que son utilisation, en particulier dans le domaine du capillaire.

On connaît des compositions épaissies, notamment des gels capillaires, qui sont généralement appliquées sur les cheveux avant de mettre en forme la coiffure. Pour mettre en forme et fixer la coiffure, on effectue ensuite un brushing ou une mise en plis. Ces compositions épaissies, notamment ces gels, peuvent contenir des résines polymères.

Toutefois, elles présentent souvent l'inconvénient d'altérer les propriétés cosmétiques des cheveux. Ainsi, les cheveux peuvent devenir rêches, difficiles à démêler, perdre leur toucher et leur aspect agréables ou encore manquer de corps. On recherche donc de telles compositions cosmétiques capillaires procurant de bonnes propriétés cosmétiques, notamment en terme de démêlage, de douceur et de toucher.

En outre, les compositions cosmétiques capillaires épaissies présentent également l'inconvénient majeur de donner lieu à un effet de poudrage. Au sens de la présente invention, on entend par *« poudrage* », l'aptitude du matériau obtenu par séchage de la composition capillaire à former une poudre après son application sur les cheveux.

Les milieux cosmétiques aqueux sont, en général, gélifiés par des polymères hydrophiles réticulés. Il est difficile de réaliser des gels cosmétiques aqueux qui auraient, après le séchage sur les cheveux, une bonne résistance à un climat humide.

Il existe donc un besoin de trouver des compositions cosmétiques, notamment capillaires épaissies, notamment des gels capillaires, qui ne présentent pas l'ensemble des inconvénients indiqués ci-dessus, et qui, en particulier résistent à un climat humide.

Pour résoudre ce problème, l'invention propose l'utilisation en cosmétique de petites molécules chirales à fonctions amides et à chaînes grasses, contenant des fonctions ioniques. Ces molécules sont décrites dans la littérature, et notamment dans les publications suivantes: Chemistry Letters 2002, (7), 704-705; New Journal of Chemistry 2002, 26 (7), 817-818; Chemical Communications (Cambridge, United Kingdom) 2002, (8), 884-885, en association avec au moins un polymère bénéfique.

La Demanderesse a maintenant découvert qu'après le séchage sur les cheveux, ces gels forment des dépôts ayant une excellente tenue à l'humidité.

L'invention a pour objet une composition cosmétique, comprenant, en milieu aqueux ou eau+solvant, au moins un polymère bénéfique pour la chevelure et au moins un composé moléculaire épaississant ionique non phosphoré, de poids moléculaire inférieur à 2 000 g/mol et comprenant au moins:
- deux groupements amide,
- un carbone asymétrique,
- une chaîne grasse comportant au moins huit carbones.

Par *"composé-moléculaire",* on entend, au sens de la présente invention, tout composé dont le squelette ne résulte pas d'une polymérisation. Ceci n'exclut pas que le composé puisse contenir un ou plusieurs groupement polyoxyalkylénés ou polyoxyglycérilés.

Par *"molécule ionique",* on entend, au sens de la présente invention, une molécule comprenant au moins un atome d'azote quaternisé ou un groupement choisi parmi les groupement CO2M ou SO3M, M désignant un atome d'hydrogène ou un ion issu d'un métal alcalin ou alcalino-terreux ou un ion issu d'une amine organique.

### COMPOSE MOLECULAIRE EPAISSISSANT

Conformément à l'invention, le composé moléculaire épaississant ionique, non phosphoré, est de poids moléculaire inférieur à 2 000 g/mol et il comprend au moins:
- deux groupements amide,
- un carbone asymétrique,
- une chaîne grasse comportant au moins huit carbones.

Plus avantageusement encore, la chaîne grasse comporte entre 8 et 30 atomes de carbone, et plus préférentiellement entre 8 et 22 atomes de carbone.

De préférence, le composé moléculaire épaississant est cationique et est de poids moléculaire inférieur à 1000g/mol, et de préférence, inférieur à 800 g/mol.

Avantageusement, au moins une chaîne grasse est liée directement au groupement amide, et en particulier, au moins une chaîne grasse est liée au groupement amide par une fonction carbonyle. Plus préférentiellement, le composé moléculaire épaississant contient au moins deux chaînes grasses et, encore plus préférentiellement, au moins une chaîne grasse fait partie d'un groupement ester.

Selon on mode préféré de l'invention, le composé moléculaire épaississant contient au moins deux enchaînements -CONH-.

En particulier, le composé épaississant est présent dans le milieu aqueux à une concentration en poids comprise entre 0,1 et 60 %, et de préférence entre 0,25 et 25 % en poids, par rapport au poids total de la composition cosmétique.

Préférentiellement, la composition forme un film après séchage. Dans un mode préféré, le film est insoluble dans l'eau pure à température ambiante. Il peut, par contre, être soluble, à pH acide ou alcalin.

En tant que composé moléculaire épaississant, on peut citer le bromure de 1-(11-(((1 S)-1-éthoxycarbonyl-5-((1-oxododécyl)amino)pentyl)amino)-11-oxoundécyl) pyridinium.

### POLYMÈRE BENEFIQUE

Les compositions capillaires de la présente invention contiennent au moins un polymère bénéfique pour la chevelure.

Par *''polymère bénéfique",* on entend au sens de la présente invention, tout polymère permettant d'améliorer les propriétés cosmétiques du support sur lequel il est appliqué, ou d'apporter de nouvelles propriétés cosmétiques audit support. Les principales propriétés cosmétiques à considérer sont la souplesse, la brillance, la photoprotection pour l'ensemble des supports, et plus particulièrement pour les cheveux, le démêlage, le maintien ou l'effet coiffant.

Ce polymère peut être de nature cationique, anionique, non-ionique ou amphotère.
Les polymères cationiques sont choisis par exemple parmi ceux décrits dans les demandes de brevet EP 0 337 354, FR 2 270 846, FR 2 383 660, FR 2 598 611, FR 2 470 596 et FR 2 519 863.
Les polymères cationiques préférés sont choisis parmi ceux qui contiennent des motifs comportant des groupements amines primaires, secondaires, tertiaires et/ou quaternaires qui font partie de la chaîne macromoléculaire principale, ou bien sont portés par des groupes latéraux directement reliés à celle-ci.
Parmi les polymères cationiques, on peut citer plus particulièrement les polymères du type polyamine, polyaminoamide et polyammonium quaternaire. Il s'agit de produits connus.

Les polymères du type polyamine, polyaminoamide et polyammonium quaternaire, que l'on peut utiliser dans les compositions de la présente invention, sont ceux décrits dans les brevets FR 2 505 348 et FR 2 542 997. Parmi ces polymères, on peut citer en particulier :
(1) les homopolymères ou copolymères d'esters ou d'amides acryliques ou méthacryliques à fonction aminée,
   Les copolymères de la famille (1) peuvent contenir en outre un ou plusieurs motifs dérivant de comonomères choisis dans la famille des acrylamides, méthacrylamides, diacétone-acrylamides, acrylamides et méthacrylamides substitués sur l'atome d'azote par des groupes alkyle inférieur en C₁₋₄, des groupes dérivés des acides acryliques ou méthacryliques ou de leurs esters, de vinyllactames tels que la vinylpyrrolidone ou le vinylcaprolactame, d'esters vinyliques.
   Parmi ces copolymères de la famille (1), on peut citer en particulier :
   - les copolymères d'acrylamide et de méthacrylate de diméthylaminoéthyle quaternisé au sulfate de diméthyle ou avec un halogénure de diméthyle, tels que celui vendu sous la dénomination HERCOFLOC^{®} par la société HERCULES,
   - les copolymères d'acrylamide et de chlorure de méthacryloyloxyéthyl-triméthylammonium décrits, par exemple, dans la demande de brevet EP-A-080976 et vendus sous la dénomination BINAQUAT^{®} P 100 par la société CIBA GEIGY,
   - le copolymère d'acrylamide et de méthosulfate de méthacryloyloxyéthyl-triméthylammonium vendu sous la dénomination RETEN^{®} par la société HERCULES,
   - les copolymères vinylpyrrolidone/acrylate ou méthacrylate de dialkylaminoalkyle quaternisés ou non, tels que les produits vendus sous la dénomination GAFQUAT^{®} par la société ISP comme, par exemple, GAFQUAT^{®} 734 ou GAFQUAT^{®} 755, ou bien les produits dénommés COPOLYMER 845, 958 et 937. Ces polymères sont décrits en détail dans les brevets FR 2 077 143 et FR 2 393 573,
   - les terpolymères méthacrylate de diméthylaminoéthyle/vinylcaprolactame/vinylpyrrolidone tels que le produit vendu sous la dénomination GAFFIX^{®} VC 713 par la société ISP,
   - les copolymères vinylpyrrolidone/méthacrylamidopropyl-di-méthylamine commercialisés notamment sous la dénomination STYLEZE^{®} CC 10 par ISP, et
   - les copolymères vinylpyrrolidone/méthacrylamide de diméthylaminopropyle quaternisé, tels que le produit vendu sous la dénomination GAFQUAT^{®} HS 100 par la société ISP.
(2) Les dérivés d'éthers de cellulose comportant des groupements ammonium quaternaires décrits dans le brevet FR 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium.
(3) Les dérivés cationiques de la cellulose tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyltriméthylammonium, de méthacrylamidopropyl-triméthylammonium, de diméthyldiallylammonium.
   Les produits commercialisés répondant à cette définition sont plus particulièrement les produits vendus sous la dénomination Celquat^{®} L 200 et Celquat^{®} H 100 par la Société National Starch.
(4) Les polysaccharides cationiques décrits plus particulièrement dans les brevets US 3 589 578 et US 4 031 307, tels que les gommes de guar contenant des groupements cationiques trialkylammonium. On utilise, par exemple, des gommes de guar modifiées par un sel, par exemple le chlorure, de 2,3-époxypropyltriméthylammonium.
   De tels produits sont commercialisés notamment sous les dénominations commerciales de JAGUAR^{®} C13 S, JAGUAR^{®} C 15, JAGUAR^{®} C 17 ou JAGUAR^{®} C162 par la société MEYHALL.
(5) Les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets FR 2 162 025 et FR 2 280 361.
(6) Les polyaminoamides solubles dans l'eau, préparés en particulier par polycondensation d'un composé acide avec une polyamine. Ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé, l'agent réticulant étant utilisé dans des proportions allant de 0,025 à 0,35 mole par groupement amine du polyaminoamide. Ces polyaminoamides peuvent être alkylés ou, s'ils comportent une ou plusieurs fonctions amines tertiaires, quaternisées. De tels polymères sont notamment décrits dans les brevets FR 2 252 840 et FR 2 368 508.
(7) Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyl-dialkylènetriamine dans lesquels le groupe alkyle comporte de 1 à 4 atomes de carbone et désigne de préférence un groupe méthyle, éthyle ou propyle, et le groupe alkylène comporte de 1 à 4 atomes de carbone, et désigne de préférence le groupe éthylène. De tels polymères sont notamment décrits dans le brevet FR 1 583 363.
   Parmi ces dérivés, on peut citer plus particulièrement les polymères acide adipique/diméthylaminohydroxypropyl-diéthylène-triamine vendus sous la dénomination Cartaretine^{®} F, F4 ou F8 par la société Sandoz.
(8) Les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone, le rapport molaire de la polyalkylène-polylamine à l'acide dicarboxylique étant compris entre 0,8:1 et 1,4:1. Le polyaminoamide résultant de cette réaction est ensuite amené à réagir avec l'épichlorhydrine dans un rapport molaire d'épichlorhydrine par rapport au groupement amine secondaire du polyaminoamide compris entre 0,5:1 et 1,8:1. De tels polymères sont notamment décrits dans les brevets US 3 227 615 et US 2 961 347.
   Des polymères de ce type sont en particulier commercialisés sous la dénomination Hercosett^{®} 57 par la société Hercules Inc. ou bien sous la dénomination de PD 170 ou Delsette^{®} 101 par la société Hercules dans le cas du copolymère d'acide adipique/époxypropyl-diéthylène-triamine.
(9) Les cyclopolymères d'alkyldiallylamine ou de dialkyldiallylammonium tels que les homopolymères ou copolymères comportant, comme constituant principal de la chaîne, des motifs répondant aux formules (Va) ou (Vb) : 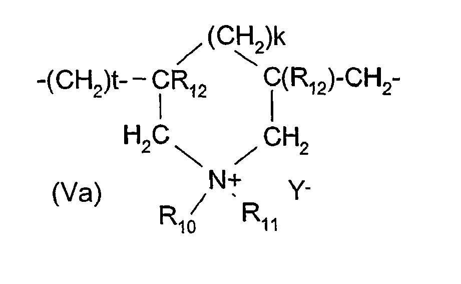 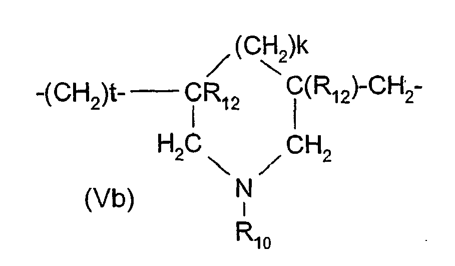 dans lesquelles
   k et t sont égaux à 0 ou 1, la somme k + t étant égale à 1 ;
   R₁₂ désigne un atome d'hydrogène ou un groupe méthyle ;
   R₁₀ et R₁₁, indépendamment l'un de l'autre, désignent un groupement alkyle ayant de 1 à 6 atomes de carbone, un groupement hydroxyalkyle en C₁₋₅, un groupement amidoalkyle inférieur en C₁-C₄, ou bien R₁₀ et R₁₁ peuvent désigner conjointement avec l'atome d'azote auquel ils sont rattachés, des groupements hétérocycliques, tels que pipéridinyle ou morpholinyle ;
   Y⁻ est un anion tel que bromure, chlorure, acétate, borate, citrate, tartrate, bisulfate, bisulfite, sulfate, phosphate.
   Ces polymères sont notamment décrits dans le brevet FR 2 080 759 et dans son certificat d'addition 2 190 406.
   Parmi les polymères définis ci-dessus, on peut citer plus particulièrement l'homopolymère de chlorure de diméthyldiallyl-ammonium vendu sous la dénomination MERQUAT^{®} 100 par la société CALGON (et ses homologues de faibles masses moléculaires moyenne en poids) et les copolymères de chlorure de diallyldiméthylammonium et d'acrylamide commercialisés sous la dénomination MERQUAT^{®} 550.
(10) Les polymères de diammonium quaternaire contenant des motifs récurrents répondant à la formule (VI) : 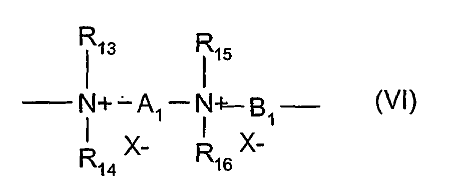 dans laquelle :
   R₁₃, R₁₄, R₁₅ et R₁₆, identiques ou différents, représentent des groupes aliphatiques, alicycliques ou arylaliphatiques contenant de 1 à 20 atomes de carbone ou des groupes hydroxyalkyle aliphatiques inférieurs, ou bien R₁₃, R₁₄, R₁₅ et R₁₆, ensemble ou séparément, forment avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁₋₆, linéaire ou ramifié, substitué par un groupement nitrile, ester, acyle, amide ou -CO-O-R₁₇-D ou -CO-NH-R₁₇-D où R₁₇ est un groupe alkylène et D un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement :

      -(CH₂)ₙ-CO-D-OC-(CH₂)ₙ-

      dans lequel D désigne :
      a) un reste de glycol de formule -O-Z-O-, où Z désigne un groupe hydrocarboné linéaire ou ramifié, ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂-;
      d) un groupement uréylène de formule -NH-CO-NH-.
      De préférence, X⁻ est un anion tel que le chlorure ou le bromure.
      Ces polymères ont une masse moléculaire moyenne en nombre généralement comprise entre 1000 et 100000.
      Des polymères de ce type sont notamment décrits dans les brevets FR 2 320 330, FR 2 270 846, FR 2 316 271, FR 2 336 434 et FR 2 413 907 et les brevets US 2 273 780, US 2 375 853, US 2 388 614, US 2 454 547, US 3 206 462, US 2 261 002, US 2 271 378, US 3 874 870, US 4 001 432, US 3 929 990, US 3 966 904, US 4 005 193, US 4 025 617, US 4 025 627, US 4 025 653, US 4 026 945 et US 4 027 020.
      On peut utiliser plus particulièrement les polymères qui sont constitués de motifs récurrents répondant à la formule : 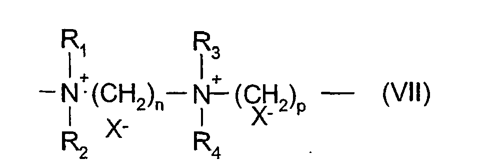
   dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (VII) particulièrement préféré est celui pour lequel R₁, R₂, R₃ et R₄ représentent un groupe méthyle et n=3, p=6 et X=Cl, dénommé chlorure d'hexadiméthrine (CTFA).
(11) Les polymères de polyammonium quaternaire,
   On peut citer parmi ceux-ci, par exemple, les produits Mirapol^{®} A 15, Mirapol^{®} AD1, Mirapol^{®} AZ1 et Mirapol^{®} 175 vendus par la société Miranol.
(12) Les polymères quaternaires de vinylpyrrolidone et de vinylimidazole tels que, par exemple, les produits commercialisés sous les dénominations Luviquat^{®} FC 905, FC 550 et FC 370 par la société B.A.S.F. On peut citer notamment les copolymères de vinylpyrrolidone et de chlorure de méthylvinylimidazolium.
(13) Les polyamines comme le Polyquart^{®} H vendu par HENKEL, référencées sous le nom de POLYETHYLENEGLYCOL (15) TALLOW POLYAMINE dans le dictionnaire CTFA.
(14) Les polymères réticulés ou non réticulés de sels de méthacryloyloxyalkyl(C₁₋₄) trialkyl(C₁₋₄)ammonium, tels que les polymères obtenus par homopolymérisation du méthacrylate de diméthylaminoéthyle quaternisé par le chlorure de méthyle, ou par copolymérisation de l'acrylamide et de méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bisacrylamide. On peut plus particulièrement utiliser un copolymère réticulé acrylamide/chlorure de méthacryloyloxy-éthyl-triméthylammonium (20/80 en poids) sous forme de dispersion contenant 50 % en poids dudit copolymère dans de l'huile minérale. Cette dispersion est commercialisée sous le nom de SALCARE^{®} SC 92 par la Société ALLIED COLLOIDS. On peut également utiliser un homopolymère réticulé du chlorure de méthacryloyloxyéthyl-triméthylammonium contenant environ 50 % en poids de l'homopolymère dans de l'huile minérale ou dans un ester liquide. Ces dispersions sont commercialisées sous les noms de SALCARE^{®} SC 95 et SALCARE^{®} SC 96 par la Société ALLIED COLLOIDS.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires, les dérivés cationiques de la chitine, et les silicones aminofonctionnelles.

Parmi tous les polymères cationiques susceptibles d'être utilisés dans les compositions cosmétiques de la présente invention, on préfère les dérivés d'éthers de cellulose comportant des groupements ammonium quaternaires, tels que les produits vendus sous la dénomination JR 400 par la Société UNION CARBIDE CORPORATION, les cyclopolymères cationiques, en particulier les homopolymères ou copolymères de chlorure de diméthyldiallylammonium, vendus sous les dénominations MERQUAT^{®} 100, MERQUAT^{®} 550 et MERQUAT^{®} S par la société CALGON, les polysaccharides cationiques tels que les gommes de guar modifiées par un sel de 2,3-époxypropyl-triméthylammonium, les copolymères quaternisés de vinylpyrrolidone et de vinylimidazole, les polycondensats de polyammonium quaternaire comportant de préférence les motifs récurrents de formules (VI) et (VIII) telles qu'indiquées ci-dessus, et leurs mélanges.

Les polymères bénéfiques anioniques généralement utilisés sont des polymères comportant des groupements dérivés d'acide carboxylique, d'acide sulfonique ou d'acide phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule : 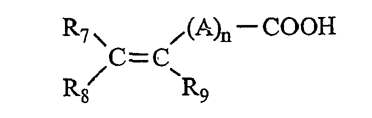 dans laquelle
n est un nombre entier de 0 à 10,
A₁ désigne un groupement méthylène, éventuellement relié à l'atome de carbone du groupement insaturé ou au groupement méthylène voisin lorsque n est supérieur à 1, par l'intermédiaire d'un hétéroatome tel que l'oxygène ou le soufre,
R₇ désigne un atome d'hydrogène, un groupement phényle ou benzyle,
R₈ désigne un atome d'hydrogène, un groupement alkyle inférieur ou carboxyle, et
R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères bénéfiques anioniques à groupements carboxyliques préférés selon l'invention sont :
A) Les homo- ou copolymères d'acide acrylique ou méthacrylique ou leurs sels et en particulier les produits vendus sous les dénominations VERSICOL^{®} E ou K par la société ALLIED COLLOID et ULTRAHOLD^{®} par la société BASF, les copolymères d'acide acrylique et d'acrylamide vendus sous la forme de sel de sodium sous les dénominations RETEN^{®} 421, 423 ou 425 par la société HERCULES, et les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylèneglycol tel que le polyéthylèneglycol, et éventuellement réticulés. De tels polymères sont décrits en particulier dans le brevet français FR 1 222 944 et la demande de brevet allemand DE 2 330 956, les copolymères de ce type comportant dans leur chaîne un motif acrylamide éventuellement N-alkylé et/ou N-hydroxyalkylé tels que décrits notamment dans les demandes de brevets luxembourgeois n° 75370 et 75371 et proposés sous la dénomination QUADRAMER^{®} par la société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE^{®} LM et les terpolymères acide méthacrylique/acrylate d'éthyle/acrylate de tertiobutyle tels que le produit commercialisé sous la dénomination LUVIMER^{®} 100 P par la société BASF.
C) Les copolymères dérivés d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés et réticulés, ou encore un autre monomère ester vinylique, allylique ou méthallylique d'un acide carboxylique α-ou β-cyclique. De tels polymères sont décrits entre autres dans les brevets français FR 1 222 944, FR 1 580 545, FR 2 265 782, FR 2 265 781, FR 1 564 110 et FR 2 439 798. Des produits commerciaux entrant dans cette classe sont les résines 28-29-30, 26-13-14 et 28-13-10 commercialisée par la société NATIONAL STARCH.
D) Les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n° 2,047,398, 2,723,248 et 2,102,113, et le brevet GB 839 805. Des produits commerciaux sont notamment ceux vendus sous les dénominations GANTREZ^{®} AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.
   Ces polymères sont par exemple décrits dans les brevets français FR 2 350 384 et FR 2 357 241 de la demanderesse.
E) Les polyacrylamides comportant des groupements carboxylates.
   Les polymères comprenant les groupements sulfoniques sont des polymères comportant des motifs vinylsulfonique, styrène-sulfonique, naphtalène-sulfonique ou acrylamido-alkylsulfonique.
   Ces polymères peuvent être notamment choisis parmi :
   - les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
   - les sels de l'acide polystyrène-sulfonique tels que les sels de sodium vendus par exemple sous les dénominations FLEXAN^{®} 130 et FLEXAN^{®} 500 par la société NATIONAL STARCH. Ces composés sont décrits dans le brevet FR 2 198 719.
   - les sels d'acides polyacrylamide-sulfoniques tels que ceux mentionnés dans le brevet US 4,128,631, et plus particulièrement l'acide polyacrylamidoéthylpropanesulfonique vendu sous la dénomination COSMEDIA POLYMER^{®} HSP 1180 par la société HENKEL.

Les polymères bénéfiques amphotères utilisables dans les compositions capillaires de la présente invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère comportant un ou plusieurs groupements carboxyliques ou sulfoniques. Les polymères bénéfiques amphotères peuvent également comporter des motifs zwitterioniques de type carboxybétaïne ou sulfobétaïne.
Il peut également s'agir de polymères à chaîne principale cationique comportant des groupements amine primaire, secondaire, tertiaire ou quaternaire, parmi lesquels au moins un porte, par l'intermédiaire d'un radical hydrocarboné, un groupement acide carboxylique ou acide sulfonique. Les polymères bénéfiques amphotères peuvent encore avoir une chaîne anionique dérivée d'acides carboxyliques α,β-insaturés dont l'un des groupements carboxyle a été amené à réagir avec une polyamine comportant un ou plusieurs groupements amine primaire.

Les polymères bénéfiques amphotères répondant à la définition donnée ci-dessus sont choisis notamment parmi les polymères suivants :
(1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloroacrylique, et d'un monomère basique dérivé d'un composé vinylique substitué contenant au moins un atome basique, tel que plus particulièrement les méthacrylate et acrylate de dialkylaminoalkyle, les dialkylaminoalkyl-méthacrylamide et acrylamide. De tels composés sont décrits dans le brevet américain US 3,836,537.
(2) les polymères comportant des motifs dérivant :
   (a) d'au moins un monomère choisi parmi les acrylamides ou les méthacrylamides substitués sur l'atome d'azote par un groupe alkyle,
   (b) d'au moins un comonomère acide contenant un ou plusieurs groupements carboxyliques réactifs, et
   (c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.
      Les acrylamides ou méthacrylamides N-substitués sont notamment les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
      Les comonomères basiques préférés sont des méthacrylates d'aminoéthyle, de butylaminoéthyle, de N,N'-diméthylaminoéthyle, de N-tertio-butylaminoéthyle.
      On utilise particulièrement les copolymères dont la dénomination CTFA (4^{ème} Ed., 1991) est Octylacrylamide/acrylates/butylaminoethyl-methacrylate copolymer, tels que les produits vendus sous la dénomination AMPHOMER^{®} ou LOVOCRYL^{®} 47 par la société NATIONAL STARCH.
(3) les polyaminoamides réticulés et alcoylés partiellement ou totalement dérivant de polyaminoamides de formule générale :

   -(CO-R₁₀-CO-Z-)-

   dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   - dans les proportions de 60 à 100 % en moles, le groupe

      -NH-[(CH₂)ₓ-NH]ₚ-

      où x = 2 et p = 2 ou 3, ou bien x = 3 et p = 2
      ce groupe dérivant de la diéthylène-triamine, de la triéthylène-tétraamine ou de la dipropylène-triamine;
   - dans les proportions de 0 à 40 % en moles, le groupe

      -NH-[(CH₂)ₓ-NH]ₚ-

      où x = 2 et p = 1 et qui dérive de l'éthylène-diamine, ou le groupe dérivant de la pipérazine : 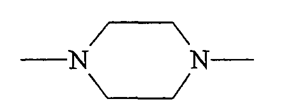
   - dans les proportions de 0 à 20 % en moles, le groupe

      -NH-(CH₂)₆-NH-
   dérivant de l'hexaméthylènediamine,
   ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et alcoylés par action d'acide acrylique, d'acide chloracétique ou d'une alcane-sultone ou de leurs sels.
   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'alcoylation sont de préférence la propane- ou la butane-sultone, les sels des agents d'alcoylation sont de préférence les sels de sodium ou de potassium.
(4) les polymères comportant des motifs zwittérioniques de formule : 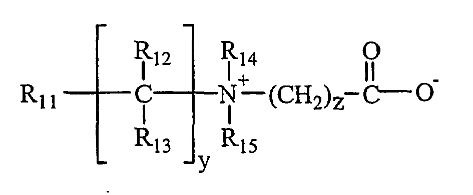 dans laquelle R₁₁ désigne un groupement insaturé polymérisable tel qu'un groupement acrylate, méthacrylate, acrylamide ou méthacrylamide, y et z représentent chacun un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthylcarboxyméthylammonioéthylméthacrylate de méthyle, tels que le produit vendu sous la dénomination DIAFORMER^{®} Z301 par la société SANDOZ.
(5) les polymères dérivés du chitosane comportant des motifs monomères répondant aux formules suivantes : 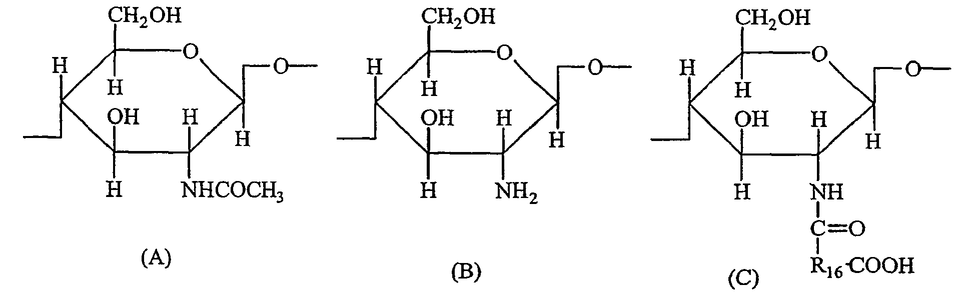 le motif (A) étant présent dans des proportions comprises entre 0 et 30%, le motif (B) dans des proportions comprises entre 5 et 50% et le motif (C) dans des proportions comprises entre 30 et 90%, étant entendu que dans ce motif (C), R₁₆ représente un groupe de formule : 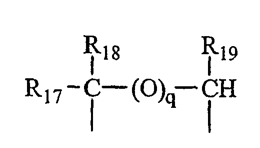 dans laquelle si q = 0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q = 1, R₁₇, R₁₈ et R₁₉ représentent chacun un atome d'hydrogène, ainsi que les sels formés par ces composés avec des bases ou des acides.
(6) Les polymères obtenus par N-carboxylation du chitosane tels que le N-carboxyméthylchitosane ou le N-carboxybutylchitosane vendu sous la dénomination EVALSAN^{®} par la société JAN DEKKER.
(7) Les polymères décrits dans le brevet français FR 1 400 366 et répondant à la formule 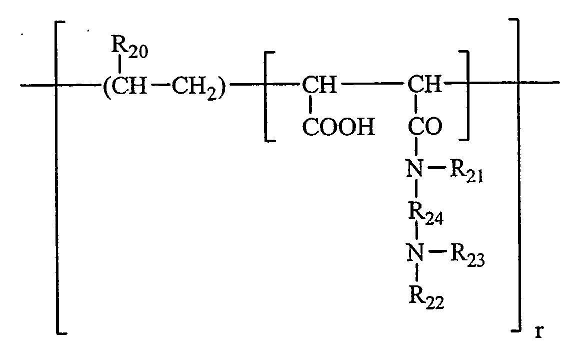 dans laquelle R₂₀ représente un atome d'hydrogène ou un groupe CH₃O, CH₃CH₂O ou phényle, R₂₁ désigne un atome d'hydrogène ou un groupe alkyle inférieur tel que méthyle et éthyle, R₂₂ désigne un atome d'hydrogène ou un groupe alkyle inférieur en C₁-C₆ tel que méthyle et éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement
   -CH₂-CH₂-, -CH₂-CH₂-CH₂-, -CH₂-CH(CH₃)-, et R₂₂ ayant les significations mentionnées ci-dessus.
(8) Les polymères amphotères du type -D-X-D-X choisis parmi:
   (a) les polymères obtenus par action de l'acide chloroacétique ou le chloroacétate de sodium sur les composés comportant au moins un motif de formule :

      -D-X-D-X-D-

      où D désigne un groupe 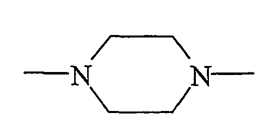 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   (b) Les polymères de formule :

      -D-X-D-X-
   où D désigne un groupe 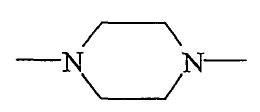 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloroacétate de soude.
(9) les copolymères alkyl(C₁-C₅)vinyléther/anhydride maléique modifiés partiellement par semi-amidification avec une N,N-dialkylaminoalkylamine telle que la N,N-diméthyaminopropylamine ou par semi-estérification avec une N,N-dialcanolamine. Ces copolymères peuvent également comporter d'autres comonomères vinyliques tels que le vinylcaprolactame.

Les polymères bénéfiques non-ioniques sont choisis, par exemple, parmi :
(a) la vinylpyrrolidone,
(b) les copolymères de vinylpyrrolidone et d'acétate de vinyle,
(c) les polyalkyloxazolines telles que les polyéthyloxazolines proposées par la société DOW CHEMICAL sous les dénominations PEOX^{®} 50 000, PEOX^{®} 200 000 et PEOX^{®} 500 000,
(d) les homopolymères d'acétate de vinyle tels que le produit proposé sous la dénomination APPRETAN^{®} EM par la société HOECHST ou le produit proposé sous le nom de RHODOPAS^{®} A 012 par la société RHONE POULENC,
(e) les copolymères d'acétate de vinyle et d'ester acrylique tels que le produit proposé sous le nom de RHODOPAS^{®} AD 310 de la société RHONE POULENC,
(f) les copolymères d'acétate de vinyle et d'éthylène tels que le produit proposé sous le nom de APPRETAN^{®} TV par la société HOECHST,
(g) les copolymères d'acétate de vinyle et d'ester maléique, par exemple, de maléate de dibutyle tels que le produit proposé sous le nom de APPRETAN^{®} MB EXTRA par la société HOECHST,
(h) les copolymères de polyéthylène et d'anhydride maléique,
(i) les poly(acrylate d'alkyle) et poly(méthacrylate d'alkyle) tels que le produit proposé sous la dénomination MICROPEARL^{®} RQ 750 par la société MATSUMOTO ou le produit proposé sous la dénomination LUHYDRAN^{®} A 848 S par la société BASF,
(j) les copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous les dénominations ACRONAL^{®} 601, LUHYDRAN^{®} LR 8833 ou 8845, par la société HOECHST sous les dénominations APPRETAN^{®} N9212 et N9213 ;
(k) les copolymères d'acrylonitrile et d'un monomère non ionique choisis, par exemple, parmi le butadiène et les (méth)acrylates d'alkyle ; on peut citer les produits proposés sous les dénominations NIPOL^{®} LX 531 B par la société NIPPON ZEON ou ceux proposés sous la dénomination CJ 0601 B par la société ROHM & HAAS,
(l) les polyamides tels que le produit ESTAPOR^{®} LO 11 proposé par la société RHONE POULENC,
(m) les gommes de guar non ioniques chimiquement modifiées ou non modifiées. Les gommes de guar non modifiées sont par exemple les produits vendus sous la dénomination VIDOGUM^{®} GH 175 par la société UNIPECTINE et sous la dénomination JAGUAR^{®} C par la société MEYHALL. Les gommes de guar modifiées sont de préférence modifiées par des groupements hydroxyalkyle en C₁₋₆, de préférénce par des groupements hydroxyméthyle, hydroxyéthyle, hydroxypropyle et hydroxybutyle. De telles gommes de guar non ioniques éventuellement modifiées par des groupes hydroxyalkyle sont par exemple vendues sous les dénominations commerciales JAGUAR^{®} HP8, JAGUAR^{®} HP60, JAGUAR^{®} HP120, JAGUAR^{®} DC 293 et JAGUAR^{®} HP 105 par la société MEYHALL, ou sous la dénomination GALACTOSOL^{®} 4H4FD2 par la société AQUALON.

On peut également utiliser, en tant que polymères bénéfiques, des polymères filmogènes de type silicone greffée comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037.

Ces polymères peuvent être anioniques, cationiques, non-ioniques ou amphotères, mais sont de préférence anioniques ou non-ioniques.

De tels polymères sont par exemple les copolymères susceptibles d'être obtenus par polymérisation radicalaire à partir du mélange de monomères formé
a) de 50 à 90 % en poids d'acrylate de tertiobutyle,
b) de 0 à 40 % en poids d'acide acrylique,
c) de 5 à 40 % en poids d'un macromère siliconé de formule 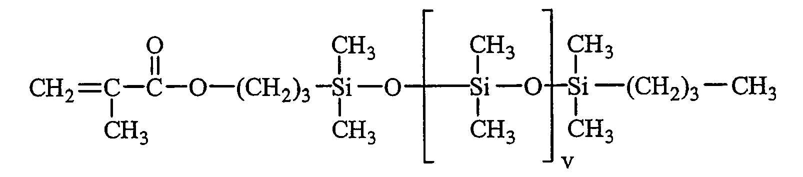
où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut également utiliser des polymères contenant des motifs uréthane. Ces polyuréthanes peuvent être fonctionnalisés ou non, siliconés ou non, cationiques, non-ioniques, anioniques ou amphotères. Les polyuréthanes particulièrement visés sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et dans les demandes EP 0 619 111 de la société National Starch. Comme polyuréthanes convenant particulièrement bien à la présente invention, on peut citer les produits commercialisés sous les dénominations LUVISET PUR^{®} et LUVISET^{®} Si PUR par la société BASF.

Les polymères bénéfiques des compositions de l'invention peuvent être solubilisés dans les compositions sous forme de dispersions.

Les polymères bénéfiques, c'est-à-dire fixants et/ou conditionneurs sont présents dans les compositions capillaires de la présente invention en une quantité suffisante pour obtenir l'effet cosmétique obtenu.

Cette quantité est généralement comprise entre 0,01 % et 20 % en poids, de préférence entre 0,1 et 10 % en poids.

Le milieu aqueux cosmétiquement acceptable peut être constitué uniquement d'eau ou d'un mélange d'eau et d'un ou de plusieurs solvants cosmétiquement acceptables miscibles avec l'eau tels que les alcools inférieurs en C₁-C₄, en particulier l'éthanol, l'isopropanol, le tertio-butanol et le n-butanol.

Les compositions selon l'invention peuvent également contenir des additifs cosmétiques et/ou des adjuvants de formulation tels que les agents tensioactifs anioniques, cationiques, amphotères ou non-ioniques, les agents nacrants, les agents opacifiants, les pigments et colorants, les huiles, les cires dont les céramides, les filtres UV organiques ou minéraux, les agents anti-radicalaires, les vitamines, les protéines, les agents anti-pelliculaires, les agents plastifiants, les agents d'ajustement et de fixation du pH, les agents anti-oxydants, les agents conservateurs, les précurseurs de colorants capillaires et les agents oxydants.

L'invention concerne également l'utilisation d'une composition selon l'invention, pour améliorer les propriétés cosmétiques des cheveux.

L'homme de métier veillera à choisir les éventuels additifs et leur quantité de manière à ce qu'ils ne nuisent pas aux propriétés intéressantes des compositions de la présente invention.

Les compositions peuvent se présenter sous la forme de fluides épaissis, de gels, de crèmes, de mousses, d'émulsions E/H, H/E ou d'émulsions multiples.

Les compositions de l'invention peuvent être appliquées sur les cheveux, la peau, les cils, les sourcils et les ongles.

Elles peuvent être utilisées, par exemple, comme shampooings, soins rincés ou non rincés, masques de soin profond, lotions ou crèmes de traitement du cuir chevelu et de la peau.

On les emploie tout particulièrement pour les cheveux.

Il existe différents modes d'application des compositions sur les cheveux. On peut citer ceux-ci:
Application 1 : la composition est appliquée sur les cheveux sous forme de gel ou de solution dans une composition capillaire contenant de l'eau, de l'alcool ou un autre solvant cosmétiquement acceptable ou leur mélange.
Application 2 : la composition est appliquée sur les cheveux sous forme de solution dans une composition capillaire contenant un ou plusieurs solvants cosmétiquement acceptables. L'eau est appliquée en deuxième temps, pour former un gel.
Application 3 : la composition est appliquée sur les cheveux sous forme de gel ou de solution dans une composition capillaire contenant de l'eau, de l'alcool ou un autre solvant cosmétiquement acceptable ou leur mélange. Après séchage, le dépôt formé sur les cheveux est éliminé par application:
   - d'eau chaude
   - d'une solution aqueuse à pH acide ou basique
   - d'un solvant ou d'un mélange de plusieurs solvants.

Ces compositions peuvent être conditionnées dans un dispositif aérosol en présence d'un ou de plusieurs agents propulseurs. Ces agents propulseurs sont de préférence choisis parmi le diméthyléther, les alcanes en C₃₋₅, le 1,1-difluoroéthane, les mélanges de diméthyléther et d'alcanes en C₃₋₅ et les mélanges de 1,1-difluoroéthane et de diméthyléther et/ou d'alcanes en C₃₋₅.

La présente invention est illustrée ci-après à l'aide d'un exemple.

### EXEMPLE:

On réalise un gel aqueux contenant 0.8 % en matière active de 1-(11-(((1S)-1-éthoxycarbonyl-5-((1-oxododécyl)amino)pentyl)amino)-11-oxoundécyl) pyridinium et 0.2 % en matière active de Jaguar HP 105 ^{®} commercialisé par Meyhall (gomme de guar non ionique).

Après l'application sur cheveux, on a remarqué que ce gel donne une tenue à l'humidité supérieure à celle du Carbopol Ultrez 10 ^{®} commercialisé par Noveon (poly acide acrylique réticulé) à 1 % en poids m.a. dans l'eau.

## Revendications

1. Composition cosmétique comprenant, en milieu aqueux ou eau+solvant, au moins un polymère bénéfique pour la chevelure et au moins un composé moléculaire épaississant ionique non phosphoré, de poids moléculaire inférieur à 2 000 g/mol, ce composé moléculaire comprenant au moins:
- deux groupements amide,
- un carbone asymétrique,
- une chaîne grasse comportant au moins huit carbones,
- un atome d'azote quaternisé ou un groupement choisi parmi les groupements CO2M ou SO3M, M désignant un atome d'hydrogène ou un ion issu d'un métal alcalin ou alcalino-terreux ou un ion issu d'une amine organique.

2. Composition selon la revendication 1, **caractérisée par le fait que** le composé moléculaire épaississant contient au moins deux enchaînements - CONH-.

3. Composition selon l'une quelconque des revendications précédentes, **caractérisée par le fait que** la chaîne grasse comporte entre 8 et 30 atomes de carbone, et plus préférentiellement entre 8 et 22 atomes de carbone.

4. Composition selon l'une quelconque des revendications précédentes, **caractérisée par le fait que** le composé moléculaire épaississant est cationique.

5. Composition selon l'une quelconque des revendications précédentes, **caractérisée par le fait que** le composé moléculaire épaississant est de poids moléculaire inférieur à 1000g/mol, et de préférence, inférieur à 800 g/mol.

6. Composition selon l'une quelconque des revendications précédentes, **caractérisée par le fait qu'**au moins une chaîne grasse est liée directement au groupement amide.

7. Composition selon l'une quelconque des revendications 1 à 5, **caractérisée par le fait qu'**au moins une chaîne grasse est liée au groupement amide par une fonction carbonyle.

8. Composition selon l'une quelconque des revendications précédentes, **caractérisée par le fait que** le composé moléculaire épaississant contient au moins deux chaînes grasses.

9. Composition selon l'une quelconque des revendications précédentes, **caractérisée par le fait qu'**au moins une chaîne grasse fait partie d'un groupement ester.

10. Composition selon l'une quelconque des revendications précédentes, **caractérisée par le fait que** le composé épaississant est présent dans le milieu aqueux à une concentration en poids comprise entre 0,1 et 60 %, et de préférence entre 0,25 et 25 % en poids, par rapport au poids total de la composition cosmétique.

11. Composition selon l'une quelconque des revendications précédentes, **caractérisée par le fait que** la composition forme un film après séchage.

12. Composition selon la revendication 11, **caractérisée par le fait que** le film est insoluble dans l'eau pure à la température ambiante.

13. Composition selon la revendication 11, **caractérisée par le fait que** le film est soluble à pH acide ou alcalin.

14. Composition selon l'une quelconque des revendications précédentes, **caractérisée par le fait que** les polymères bénéfiques pour la chevelure sont des polymères cationiques, anioniques, non-ioniques ou amphotères.

15. Composition selon la revendication 14, **caractérisée par le fait que** les polymères bénéfiques pour la chevelure cationiques sont choisis parmi les homopolymères ou copolymères d'esters ou d'amides acryliques ou méthacrylique à fonction amine, les polysaccharides à fonctions ammonium quaternaire, les polymères à motifs pipérazinyle et à motifs alkylène ou hydroxyalkylène, les polyaminoamides solubles dans l'eau, les cyclopolymères d'alkyldiallylamine ou de dialkyldiallylammonium, les polymères de diammonium quaternaire, les polymères de polyammonium quaternaire, les polymères quaternaires de vinylpyrrolidone et de vinylimidazole, les polyamines, les polymères de sels de méthacryloyloxyalkyl(C₁₋₄)trialkyl(C₁₋₄)ammonium, et les chitosanes.

16. Composition selon la revendication 14, **caractérisée par le fait que** les polymères bénéfiques pour la chevelure non-ioniques sont choisis parmi les copolymères de vinylpyrrolidone et de vinylcaprolactame, les homopolymères d'acétate de vinyle, les polyalkyloxazolines, les copolymères d'acétate de vinyle et de maléate d'alkyle, les homopolymères d'acrylate d'alkyle, les homopolymères de méthacrylate d'alkyle, les copolymères d'esters acryliques et méthacryliques, les copolymères d'acrylonitrile et d'un comonomère non-ionique, les polyamides, les polyuréthanes non-ioniques et les polymères siliconés non-ioniques.

17. Composition selon la revendication 14, **caractérisée par le fait que** les polymères bénéfiques pour la chevelure anioniques sont choisis parmi les homopolymères ou copolymères d'acide acrylique et méthacrylique ou leurs sels, les copolymères d'acide crotonique, les copolymères d'acides ou d'anhydrides monoinsaturés en C₄₋₈, les polyacrylamides à groupements carboxylate, les homopolymères ou copolymères à groupes sulfoniques, les polyuréthanes anioniques et les polymères siliconés greffés anioniques.

18. Composition selon la revendication 14, **caractérisée par le fait que** les polymères bénéfiques pour la chevelure amphotères sont choisis parmi les copolymères à motifs vinyliques acides et à motifs vinyliques basiques, les polyaminoamides réticulés et acylés, les polymères à motifs zwitterioniques, les chitosanes à groupes carboxyle, les copolymères alkyl(C₁₋₅)vinyléther/anhydride maléique modifiés par amidification partielle, les polyuréthanes amphotères et les polymères siliconés greffés amphotères.

19. Composition selon l'une quelconque des revendications précédentes, **caractérisée par le fait que** le ou les polymères bénéfiques pour la chevelure sont présents à raison de 0,01 à 20 % en poids, de préférence à raison de 0,1 à 10 % en poids.

20. Composition selon l'une quelconque des revendications précédentes, **caractérisée par le fait qu'**elle contient en outre des additifs cosmétiques et/ou des adjuvants de formulation tels que les silicones volatiles ou non volatiles, les agents tensioactifs anioniques, cationiques, amphotères ou non-ioniques, les agents nacrants, les agents opacifiants, les pigments et colorants, les huiles, les cires dont les céramides, les filtres UV organiques ou minéraux, les agents anti-radicalaires, les agents plastifiants, les vitamines, les protéines, les agents anti-pelliculaires, les agents d'ajustement et de fixation du pH, les agents anti-oxydants, les agents conservateurs, les précurseurs de colorants capillaires et les agents oxydants.

21. Composition selon l'une quelconque des revendications précédentes, **caractérisée par le fait qu'**elle se présente sous forme de lotion épaissie, de gel, de crème ou de pâte.

22. Procédé cosmétique comprenant l'application d' une composition cosmétique comprenant, en milieu aqueux ou eau+solvant, au moins un polymère bénéfique pour la chevelure et au moins un composé moléculaire épaississant ionique non phosphoré, de poids moléculaire inférieur à 2 000 g/mol, ce composé moléculaire comprenant au moins:
- deux groupements amide,
- un carbone asymétrique,
- une chaîne grasse comportant au moins huit carbones;
- un atome d'azote quaternisé ou un groupement choisi parmi les groupements CO2M ou SO3M, M désignant un atome d'hydrogène ou un ion issu d'un métal alcalin ou alcalino-terreux ou un ion issu d'une amine organique.

23. Utilisation d'une composition cosmétique comprenant, en milieu aqueux ou eau+solvant, au moins un polymère bénéfique pour la chevelure et au moins un composé moléculaire épaississant ionique non phosphoré, de poids moléculaire inférieur à 2 000 g/mol, ce composé moléculaire comprenant au moins:
- deux groupements amide,
- un carbone asymétrique,
- une chaîne grasse comportant au moins huit carbones,
- un atome d'azote quaternisé ou un groupement choisi parmi les groupements CO2M ou SO3M, M désignant un atome d'hydrogène ou un ion issu d'un métal alcalin ou alcalino-terreux ou un ion issu d'une amine organique.
pour améliorer les propriétés cosmétiques des cheveux.

## Claims

1. Cosmetic composition comprising, in an aqueous or water + solvent medium, at least one polymer that is beneficial to the hair and at least one phosphorus-free ionic thickening molecular compound, with a molecular weight of less than 2000 g/mol, this molecular compound comprising at least:
- two amide groups,
- one asymmetric carbon,
- one fatty chain comprising at least eight carbons,
- one quaternized nitrogen atom or a group chosen from the groups CO2M and SO3M, M denoting a hydrogen atom or an ion derived from an alkali metal or alkaline-earth metal, or an ion derived from an organic amine.

2. Composition according to Claim 1, **characterized in that** the thickening molecular compound contains at least two -CONH- linkages.

3. Composition according to either of the preceding claims, **characterized in that** the fatty chain contains between 8 and 30 carbon atoms and more preferably between 8 and 22 carbon atoms.

4. Composition according to any one of the preceding claims, **characterized in that** the thickening molecular compound is cationic.

5. Composition according to any one of the preceding claims, **characterized in that** the thickening molecular compound has a molecular weight of less than 1000 g/mol and preferably less than 800 g/mol.

6. Composition according to any one of the preceding claims, **characterized in that** at least one fatty chain is directly linked to the amide group.

7. Composition according to any one of Claims 1 to 5, **characterized in that** at least one fatty chain is linked to the amide group via a carbonyl function.

8. Composition according to any one of the preceding claims, **characterized in that** the thickening molecular compound contains at least two fatty chains.

9. Composition according to any one of the preceding claims, **characterized in that** at least one fatty chain forms part of an ester group.

10. Composition according to any one of the preceding claims, **characterized in that** the thickening compound is present in the aqueous medium in a weight concentration of between 0.1% and 60% and preferably between 0.25% and 25% by weight relative to the total weight of the cosmetic composition.

11. Composition according to any one of the preceding claims, **characterized in that** the composition forms a film after drying.

12. Composition according to Claim 11, **characterized in that** the film is insoluble in pure water at room temperature.

13. Composition according to Claim 11, **characterized in that** the film is soluble at acidic or alkaline pH.

14. Composition according to any one of the preceding claims, **characterized in that** the polymers that are beneficial to the hair are cationic, anionic, nonionic or amphoteric polymers.

15. Composition according to Claim 14, **characterized in that** the cationic polymers that are beneficial to the hair are chosen from homopolymers or copolymers of acrylic or methacrylic esters or amides containing an amine function, polysaccharides containing quaternary ammonium functions, polymers containing piperazinyl units and alkylene or hydroxyalkylene units, watersoluble polyamino amides, alkyldiallylamine or dialkyldiallylammonium cyclopolymers, diquaternary ammonium polymers, polyquaternary ammonium polymers, quaternary polymers of vinylpyrrolidone and of vinylimidazole, polyamines, polymers of methacryloyloxy(C₁₋₄)alkyltri(C₁₋₄)alkylammonium salts, and chitosans.

16. Composition according to Claim 14, **characterized in that** the nonionic polymers that are beneficial to the hair are chosen from copolymers of vinylpyrrolidone and of vinylcaprolactam, vinyl acetate homopolymers, polyalkyloxazolines, copolymers of vinyl acetate and of alkyl maleate, alkyl acrylate homopolymers, alkyl methacrylate homopolymers, copolymers of acrylic and methacrylic esters, copolymers of acrylonitrile and of a nonionic comonomer, polyamides, nonionic polyurethanes and nonionic silicone polymers.

17. Composition according to Claim 14, **characterized in that** the anionic polymers that are beneficial to the hair are chosen from homopolymers or copolymers of acrylic and methacrylic acid or salts thereof, crotonic acid copolymers, C₄₋₈ monounsaturated acid or anhydride copolymers, polyacrylamides containing carboxylate groups, homopolymers or copolymers containing sulfonic groups, anionic polyurethanes and anionic grafted silicone polymers.

18. Composition according to Claim 14, **characterized in that** the amphoteric polymers that are beneficial to the hair are chosen from copolymers containing acidic vinyl units and basic vinyl units, crosslinked and acylated polyamino amides, polymers containing zwitterionic units, chitosans containing carboxyl groups, (C₁₋₅)alkyl vinyl ether/maleic anhydride copolymers modified by partial amidation, amphoteric polyurethanes and amphoteric grafted silicone polymers.

19. Composition according to any one of the preceding claims, **characterized in that** the polymer(s) that is (are) beneficial to the hair is (are) present in a proportion of from 0.01% to 20% by weight and preferably in a proportion of from 0.1% to 10% by weight.

20. Composition according to any one of the preceding claims, **characterized in that** it also contains cosmetic additives and/or formulation adjuvants such as volatile or nonvolatile silicones, anionic, cationic, amphoteric or nonionic surfactants, nacreous agents, opacifiers, pigments and dyes, oils, waxes, including ceramides, organic or mineral-UV-screening agents, free-radical scavengers, plasticizers, vitamins, proteins, antidandruff agents, pH regulating and fixing agents, antioxidants, preserving agents, hair dye precursors and oxidizing agents.

21. Composition according to any one of the preceding claims, **characterized in that** it is in the form of a thickened lotion, a gel, a cream or a paste.

22. Cosmetic process comprising the application of a cosmetic composition comprising, in an aqueous or water + solvent medium, at least one polymer that is beneficial to the hair and at least one phosphorus-free ionic thickening molecular compound, with a molecular weight of less than 2000 g/mol, this molecular compound comprising at least:
- two amide groups,
- one asymmetric carbon,
- one fatty chain comprising at least eight carbons,
- one quaternized nitrogen atom or a group chosen from the groups CO2M and SO3M, M denoting a hydrogen atom or an ion derived from an alkali metal or alkaline-earth metal, or an ion derived from an organic amine.

23. Use of a cosmetic composition comprising, in an aqueous or water + solvent medium, at least one polymer that is beneficial to the hair and at least one phosphorus-free ionic thickening molecular compound, with a molecular weight of less than 2000 g/mol, this molecular compound comprising at least:
- two amide groups,
- one asymmetric carbon,
- one fatty chain comprising at least eight carbons,
- one quaternized nitrogen atom or a group chosen from the groups CO2M and SO3M, M denoting a hydrogen atom or an ion derived from an alkali metal or alkaline-earth metal, or an ion derived from an organic amine,
to improve the cosmetic properties of the hair.

## Patentansprüche

1. Kosmetische Zusammensetzung, die in einem wässrigen Medium oder Wasser + Lösungsmittel mindestens ein für das Haar vorteilhaftes Polymer und mindestens eine nicht phosphorhaltige, ionische, verdickende, molekulare Verbindung mit einer Molmasse unter 2 000 g/mol enthält, wobei die molekulare Verbindung zumindest aufweist:
- zwei Amidgruppen,
- ein asymmetrisches Kohlenstoffatom,
- eine Fettkette, die mindestens acht Kohlenstoffatome aufweist,
- ein quaternisiertes Stickstoffatom oder eine Gruppe, die unter den Gruppen CO₂M oder SO₃M ausgewählt ist, wobei M ein Wasserstoffatom oder ein Ion, das von einem Alkalimetall oder Erdalkalimetall stammt, oder ein Ion bedeutet, das von einem organischen Amin abgeleitet ist.

2. Zusammensetzung nach Anspruch 1, **dadurch gekennzeichnet, dass** die verdickende molekulare Verbindung mindestens zwei Verknüpfungen -CONH- aufweist.

3. Zusammensetzung nach einem der vorhergehenden Ansprüche, **dadurch gekennzeichnet, dass** die Fettkette 8 bis 30 Kohlenstoffatome und noch bevorzugter 8 bis 22 Kohlenstoffatome enthält.

4. Zusammensetzung nach einem der vorhergehenden Ansprüche, **dadurch gekennzeichnet, dass** die verdickende molekulare Verbindung kationisch ist.

5. Zusammensetzung nach einem der vorhergehenden Ansprüche, **dadurch gekennzeichnet, dass** die verdickende molekulare Verbindung eine Molmasse unter 1 000 g/mol und vorzugsweise unter 800 g/mol besitzt.

6. Zusammensetzung nach einem der vorhergehenden Ansprüche, **dadurch gekennzeichnet, dass** mindestens eine Fettkette direkt an eine Amidgruppe gebunden ist.

7. Zusammensetzung nach einem der Ansprüche 1 bis 5, **dadurch gekennzeichnet, dass** mindestens eine Fettkette über eine Carbonylfunktion an eine Amidgruppe gebunden ist.

8. Zusammensetzung nach einem der vorhergehenden Ansprüche, **dadurch gekennzeichnet, dass** die verdickende molekulare Verbindung mindestens zwei Fettketten aufweist.

9. Zusammensetzung nach einem der vorhergehenden Ansprüche, **dadurch gekennzeichnet, dass** mindestens eine Fettkette Teil einer Estergruppe ist.

10. Zusammensetzung nach einem der vorhergehenden Ansprüche, **dadurch gekennzeichnet, dass** die verdickende Verbindung in dem wässrigen Medium in einer Massekonzentration von 0,1 bis 60 % und vorzugsweise 0,25 bis 25 Gew.-%, bezogen auf das Gesamtgewicht der kosmetischen Zusammensetzung, enthalten ist.

11. Zusammensetzung nach einem der vorhergehenden Ansprüche, **dadurch gekennzeichnet, dass** die Zusammensetzung nach dem Trocknen einen Film bildet.

12. Zusammensetzung nach Anspruch 11, **dadurch gekennzeichnet, dass** der Film in reinem Wasser bei Raumtemperatur unlöslich ist.

13. Zusammensetzung nach Anspruch 11, **dadurch gekennzeichnet, dass** der Film bei einem alkalischen oder sauren pH-Wert löslich ist.

14. Zusammensetzung nach einem der vorhergehenden Ansprüche, **dadurch gekennzeichnet, dass** die für das Haar vorteilhaften Polymere kationische, anionische, nichtionische oder amphotere Polymere sind.

15. Zusammensetzung nach Anspruch 14, **dadurch gekennzeichnet, dass** die für das Haar vorteilhaften kationischen Polymere unter den Homopolymeren oder Copolymeren von Acryl- oder Methacrylestern oder Acryl- oder Methacrylamiden mit Aminfunktion, Polysacchariden mit quartärer Ammoniumfunktion, Polymeren mit Piperazinyleinheiten und Alkylen- oder Hydroxyalkyleneinheiten, in Wasser löslichen Polyaminoamiden, Alkyldiallylamin- oder Dialkyldiallylammonium-Cyclopolymeren, quartären Diammoniumpolymeren, quartären Polyammoniumpolymeren, quartären Polymeren von Vinylpyrrolidon und Vinylimidazol, Polyminen, Polymeren von Methacryloyloxyalkyl(C₁₋₄)trialkyl(C₁₋₄)-ammoniumpolymeren und Chitosanen ausgewählt sind.

16. Zusammensetzung nach Anspruch 14, **dadurch gekennzeichnet, dass** die für das Haar vorteilhaften nichtionischen Polymere unter den Copolymeren von Vinylpyrrolidon und Vinylcaprolactam, Homopolymeren von Vinylacetat, Polyalkyloxazolinen, Copolymeren von Vinylacetat und Alkylmaleat, Homopolymeren von Alkylacrylat, Homopolymeren von Alkylmethacrylat, Copolymeren von Acryl- und Methacrylestern, Copolymeren von Acrylnitril und einem nichtionischen Comonomer, Polyamiden, nichtionischen Polyurethanen und nichtionischen Siliconpolymeren ausgewählt sind.

17. Zusammensetzung nach Anspruch 14, **dadurch gekennzeichnet, dass** die für das Haar vorteilhaften anionischen Polymere unter den Homopolymeren oder Copolymeren von Acrylsäure und Methacrylsäure oder deren Salzen, Copolymeren von Crotonsäure, Copolymeren von einfach ungesättigten Säuren oder Anhydriden mit 4 bis 8 Kohlenstoffatomen, Polyacrylamiden mit Carboxylatgruppen, Homopolymeren oder Copolymeren mit Sulfonsäuregruppen, anionischen Polyurethanen und anionischen gepfropften Siliconpolymeren ausgewählt sind.

18. Zusammensetzung nach Anspruch 14, **dadurch gekennzeichnet, dass** die für das Haar vorteilhaften amphoteren Polymere unter den Copolymeren mit sauren Vinyleinheiten und basischen Vinyleinheiten, vernetzten und acylierten Polyaminoamiden, Polymeren mit zwitterionischen Einheiten, Chitosanen mit Carboxygruppen, Albyl(C₁₋₅)vinylether/Maleinsäureanhydrid-Copolymeren, die durch teilweise Amidierung modifiziert sind, amphoteren Polyurethanen und gepfropften amphoteren Siliconpolymeren ausgewählt sind.

19. Zusammensetzung nach einem der vorhergehenden Ansprüche, **dadurch gekennzeichnet, dass** das oder die für das Haar günstige(n) Polymer(e) in einer Menge von 0,01 bis 20 Gew.-% und vorzugsweise 0,1 bis 10 Gew.-% enthalten sind.

20. Zusammensetzung nach einem der vorhergehenden Ansprüche, **dadurch gekennzeichnet, dass** sie ferner kosmetische Zusatzstoffe und/oder Formulierungshilfsstoffe enthält, wie flüchtige oder nicht flüchtige Silicone, anionische, kationische, amphotere oder nichtionische grenzflächenaktive Stoffe, Perlglanzstoffe, Trübungsmittel, Pigmente und Farbstoffe, Öle, Wachse und darunter Ceramide, organische oder anorganische UV-Filter, Radikalfänger für freie Radikale, Weichmacher, Vitamine, Proteine, Antischuppenmittel, Stoffe, um den pH-Wert einzustellen, und Stoffe, um den pH-Wert zu halten, Antioxidantien, Konservierungsmittel, Farbstoffvorprodukte von Haarfarbmitteln und Oxidationsmittel.

21. Zusammensetzung nach einem der vorhergehenden Ansprüche, **dadurch gekennzeichnet, dass** sie als dickflüssige Lotion, Gel, Creme oder Paste vorliegt.

22. Kosmetisches Verfahren, das das Aufbringen einer kosmetischen Zusammensetzung umfasst, die in einem wässrigen Medium oder Wasser + Lösungsmittel mindestens ein für das Haar günstiges Polymer und mindestens eine nicht phosphorhaltige, ionische, verdickende molekulare Verbindung mit einer Molmasse unter 2 000 g/mol enthält, wobei diese molekulare Verbindung zumindest aufweist:
- zwei Amidgruppen,
- ein asymmetrisches Kohlenstoffatom,
- eine Fettkette, die mindestens acht Kohlenstoffatome aufweist,
- ein quaternisiertes Stickstoffatom oder eine Gruppe, die unter den Gruppen CO₂M oder SO₃M ausgewählt ist, wobei M ein Wasserstoffatom oder ein Ion, das von einem Alkalimetall oder Erdalkalimetall stammt, oder ein Ion bedeutet, das von einem organischen Amin abgeleitet ist.

23. Verwendung einer kosmetischen Zusammensetzung, die in einem wässrigen Medium oder Wasser + Lösungsmittel mindestens ein für das Haar günstiges Polymer und mindestens eine nicht phosphorhaltige ionische verdickende molekulare Verbindung mit einer Molmasse unter 2 000 g/mol enthält, wobei diese molekulare Verbindung zumindest aufweist:
- zwei Amidgruppen,
- ein asymmetrisches Kohlenstoffatom,
- eine Fettkette, die mindestens acht Kohlenstoffatome aufweist,
- ein quaternisiertes Stickstoffatom oder eine Gruppe, die unter den Gruppen CO₂M oder SO₃M ausgewählt ist, wobei M ein Wasserstoffatom oder ein Ion, das von einem Alkalimetall oder Erdalkalimetall stammt, oder ein Ion bedeutet, das von einem organischen Amin abgeleitet ist, zur Verbesserung der kosmetischen Eigenschaften der Haare.
